# FASCICULE DE BREVET EUROPEEN

(11) **EP 0 253 715 B1**
(45) Date de publication et mention de la délivrance du brevet: **30.11.1994**
(21) Numéro de dépôt: 87401573.8
(22) Date de dépôt: 06.07.1987
(51) Int. Cl.: C08H 1/06, A61L 27/00, B29D 11/00

(54) **Procédé de traitement du collagène en vue, notamment , d'en faciliter la réticulation et collagène obtenu par application dudit procédé**
Verfahren zur Behandlung von Kollagen zur Erleichterung seiner Vernetzung und das so erhaltene Kollagen
Process for the treatment of collagen in order to facilitate the crosslinking and collagen obtained by the application of this process

(30) Priorité: 11.07.1986 FR 8610160
(43) Date de publication de la demande: 20.01.1988
(73) Titulaire: PASTEUR MERIEUX SERUMS ET VACCINS, 69007 Lyon (FR); IMEDEX, F-69630 Chaponost (FR)
(72) Inventeur: Tardy, Michel, F-69005 Lyon (FR); Tayot, Jean-Louis, F-69890 La Tour De Salvagny (FR)
(74) Mandataire: Bernasconi, Jean

(56) Documents cités:
- GB-A- 915 441
- US-A- 4 164 559
- US-A- 4 223 984
- BIOCHIM. BIOPHYS. ACTA, vol. 147, 1967, pages 272-279; J. BELLO et al.: "Spontaneous cross-linking of collagen: Evidence for metal-catalyzed and oxidative reactions"
- THE JOURNAL OF BIOLOGICAL CHEMISTRY, vol. 240, no. 8, août 1965, III, pages PC3449-PC3450; W.T. BUTLER et al.: "The site of attachment of Hexose in tropocollagen"
- THE JOURNAL OF BIOLOGICAL CHEMISTRY, vol. 242, no. 5, 10 mars 1967, pages 809-812; R.B. ARONSON et al.: "The oxidation of protein-bound hydroxylysine by periodate"
- Z. NATURFORSCHUNG, vol. 12B, 1957, pages 788-791; H. ZAHN et al.: "Zur Einwirkung von Perjodat auf Kollagen und Keratin"

## Description

La présente invention a trait à un procédé de traitement du collagène en vue, notamment, d'en faciliter la réticulation, et permettant d'obtenir un collagène réticulé à la stabilité et aux caractéristiques mécaniques améliorées.

Le collagène dans ses utilisations médicales et biomédicales doit répondre à des exigences mécaniques, physico-chimiques et biologiques très rigoureuses.

Une des limitations à l'utilisation du collagène réside dans sa résistance à la biodégradabilité souvent insuffisante et dans ses caractéristiques mécaniques faibles qui en rendent la manipulation difficile.

La biodégradabilité du collagène dépend essentiellement du degré de réticulation et de la façon dont la réticulation est réalisée. Elle peut varier de quelques jours pour un collagène non tanné à plusieurs mois pour un collagène fortement réticulé. Cette réticulation permet d'introduire un certain nombre de liaisons croisées intra- et inter-chaînes, afin de diminuer la solubilité du collagène.

La réticulation du collagène peut être réalisée soit par voie chimique à l'aide d'agents tannants tels que le glutaraldéhyde ou le formaldéhyde, ou encore le diisocyanate, soit par des agents physiques tels que les radiations gamma, bêta ou ultraviolettes. Mais cette dernière méthode est lourde et parfois difficile à mettre en oeuvre et outre les réticulations, elle provoque aussi des coupures.

Le traitement par le glutaraldéhyde, qui est le traitement le plus souvent utilisé pour réticuler le collagène et qui consiste à immerger dans une solution de glutaraldéhyde des poudres, des films, des gels ou des solutions plus ou moins concentrées, présente un certain nombre d'inconvénients dans certaines applications. L'introduction de glutaraldéhyde dans une structure collagénique aqueuse (gel ou éponge) conduit à la formation de polymères de glutaraldéhyde de très haut poids moléculaire qui sont difficiles à éliminer par simple lavage et peuvent s'hydrolyser et entraîner un relargage de glutaraldéhyde ou subsister in situ et se trouver libérés après disparition de la partie collagénique.

On a également déjà réticulé du collagène traité par une oxydase spécifique mais un tel traitement est onéreux et particulièrement délicat et ne peut être mené que dans des conditions très particulières (difficulté d'éliminer la lysyl oxydase en fin de traitement).

On a déjà étudié l'action de l'acide périodique ou des périodates sur certains composants du collagène. Robert B. ARONSON et al. (Journal of Biological Chemistry, Vol. 242 n° 5, 1967, 809-812) montrent qu'il est possible de détruire une partie seulement de l'hydroxylysine d'un collagène par traitement à l'acide périodique en présence de soude. La solution de collagène reste liquide après le traitement et est destinée à être chromatographiée. William BUTLER et al., The Journal of Biologycal Chemistry Vol. 240 1965, PC 3449-50 décrit également la destruction de l'hydroxylysine par l'action de l'acide périodique sur des glycopeptides résultant d'une digestion poussée d'un collagène. L'enseignement de ces documents ne conduit pas à la réticulation des solutions de collagène.

La présente invention vise à remédier aux inconvénients précités , en proposant un procédé de traitement du collagène permettant de réticuler dans la masse et, de façon homogène, le collagène sans addition covalente de réactifs chimiques.

L'un des buts de l'invention est de fournir des gels de collagène réticulés de façon homogène et de grande stabilité.

Un autre but de l'invention est de fournir un procédé beaucoup plus souple de réticulation du collagène permettant, dans un premier temps, d'introduire une oxydation ménagée du collagène sans déclencher une réticulation importante (en jouant sur le pH : gamme plus acide) et, dans un deuxième temps, à volonté par neutralisation ou alcalinisation du pH, de déclencher une réticulation des molécules de collagène.

Un autre but de l'invention est de fournir des poudres ou des films de collagène présentant une stabilité et des caractéristiques, notamment d'insolubilité, améliorées, même à pH acide.

Un autre but encore de l'invention est de fournir des poudres ou des films présentant, en surface, des groupements réactifs permettant le couplage du collagène avec des molécules biologiquement actives.

L'invention a pour objet un procédé de réticulation du collagène en solution acqueuse, caractérisé en ce qu'il consiste à soumettre le collagène à une oxydation ménagée par une solution d'acide périodique ou de périodate de sodium, à une concentration comprise entre 10⁻¹ et 10⁻⁴ M à température ambiante, à pH acide, puis à procéder à une solidification du collagène.

Le traitement par le periodate de sodium peut être effectué, soit sur des solutions de collagène, soit sur des gels déjà constitués, soit encore sur des films ou des poudres.

Lorsque le traitement par le periodate est effectué sur des solutions collagéniques en vue de la préparation de gels, on ajoute à la solution de collagène dont la concentration en collagène est généralement comprise entre 0,1 % et 20 %, une solution d'acide periodique ou de periodate de sodium, jusqu'à l'obtention d'une concentration de 0,1 M à 0,0001 M, le pH, qui peut être compris entre 2 et 8, étant avantageusement acide dans un premier temps, puis neutre ou alcalin dans un deuxième temps, ce qui conduit rapidement à la formation d'un gel insoluble dans une très large gamme de pH, y compris à pH acide, où avant ce traitement le collagène était soluble.

Les groupements aldéhydiques en excès formés sur la molécule du collagène sont neutralisés, par example par une solution de glycocolle, d'éthanolamine et/ou d'hydroborure de sodium, ou utilisés pour le couplage de protéines, fibronectine, facteurs de croissance, glycosaminoglycanes, enzymes, agents bactéricides ou bactériostatiques, antibiotiques ou tout autre produit autorisant une meilleure biocompatibilité et une plus grande résistance à la biodégradation.

Le traitement par le periodate peut être éventuellement complété par un tannage de surface, soit par une solution de NaIO4, soit par une solution de di- ou polyaldéhyde.

Un tannage de surface est obtenu dans un deuxième temps, si nécessaire, en traitant le gel de collagène par une solution d'acide periodique ou de periodate de sodium ou par une solution de di- ou polyaldéhyde dont la concentration est comprise entre 0,1 et 0,001 M et en amenant simultanément ou dans un deuxième temps le pH à une valeur comprise entre 5 et 8 pour favoriser la réticulation des chaînes de collagène

Les solutions collagéniques traitées par l'acide periodique ou le periodate de sodium peuvent être utilisées pour préparer, par des procédés connus en soi, des fibres, des poudres, des billes ou des films etc... dont les caractéristiques mécaniques seront améliorées et auxquels pourront être associées des molécules biologiquement actives.

Comme indiqué précédemment, le traitement par l'acide periodique ou le periodate de sodium peut être également effectué sur des gels déjà constitués en vue de leur assurer une meilleure stabilité.

Il faut, à ce propos, noter que l'acide periodique ou le periodate de sodium, grâce à leur petite taille, moléculaire diffusent facilement à l'intérieur d'un gel et assurent une réticulation homogène.

Le gel est traité par une soluction d'acide periodique ou de periodate de sodium dont la concentration est avantageusement comprise entre 10⁻¹ et 10⁻⁴ M, à un pH supérieur à 5 choisi de manière à éviter une redissolution du gel avant la réaction, pendant 3 à 20 heures, à la température ambiante.

Il est également possible d'effectuer le traitement conforme à l'invention sur des films ou des poudres afin de leur assurer une plus grande stabilité ou de créer en surface des groupements réactifs pour un couplage ultérieur.

Ces films ou poudres de collagène sont traités par une solution d'acide periodique ou de ses sels dont la concentration est comprise entre 0,1 et 0,0001 M dans des conditions de pH où le film ou le matériau reste insoluble avant une deuxième étape de pH 5 à 8 qui favorise la réticulation conduisant à une insolubilisation renforcée.

D'une façon générale, on aura avantageusement recours au traitement conforme à l'invention chaque fois que l'on voudra améliorer la stabilité et les caractéristiques mécaniques d'un collagène réticulé et/ou associer le collagène avec une molécule biologiquement active.

D'autres avantages et particularités de l'invention apparaîtront à la lecture des examples suivants qui illustrent divers modes de réalisation de l'invention,

L'exemple 1 illustre la préparation d'un implant ou d'une lentille à partir d'une solution de collagène, le traitement par le periodate étant effectué dans cet exemple sur un gel déjà constitué.

L'exemple 2 illustre la préparation d'un implant ou d'une lentille à partir d'une solution de collagène, le traitement par l'acide periodique étant effectué sur la solution de collagène.

Les exemples 3 et 4 illustrent la préparation d'un implant ou d'une lentille à partir d'une solution de collagène, où le traitement de la solution de collagène par le periodate est complété par un tannage en surface par une solution de periodate de sodium ou de glutaraldéhyde.

Les exemples 5 et 6 se rapportent à la préparation d'un gel insoluble de collagène à partir d'une solution de collagène préalablement traitée par le periodate.

L'exemple 7 se rapporte à la préparation d'un gel insoluble de collagène associé à une molécule biologiquement active (fibronectine).

Les exemples 8 à 10 décrivent la préparation de fibres insolubles de collagène à partir de solutions de collagène traitées par le periodate, associées à une molécule biologiquement active (fibronectine).

L'exemple 11 est relatif à la préparation d'une poudre insoluble à partir d'une solution de collagène traitée par le periodate.

L'exemple 12 se rapporte à la préparation d'une poudre insoluble de collagène à partir d'une solution traitée par le periodate et à laquelle est associée une molécule biologiquement active (fibronectine ou glycosaminoglycane).

L'exemple 13 décrit la préparation d'un film insoluble de collagène à partir d'une solution de collagène traitée par le periodate.

L'exemple 14 illustre la préparation de billes insolubles de collagène à partir d'une solution de collagène traitée par le periodate.

Les exemples 15 et 16 se rapportent à la préparation de gels, de films, de fibres ou de billes insolubles à partir d'une solution de collagène traitée par le periodate, et associés à une molécule biologiquement active (glycosaminoglycane).

L'exemple 17 illustre la mise en oeuvre du traitement par le periodate sur une poudre de collagène.

### EXEMPLE 1

Une solution acide de collagène humain placentaire enrichie en type IV à 15 % en eau distillée est préparée par le procédé suivant :

300 kg de placenta sont broyés sous forme congelée pour donner des morceaux de quelques cm3. Le broyat est ensuite mélangé à 300 l de solution aqueuse contenant 8 % d'éthanol, 6 g/l de NaCl et 10 kg de cellulose. Après agitation à 10°C, l'ensemble est soumis à une opération de pressage avec un pressoir MABILLE pour séparer le sang du tissu placentaire. On obtient 102 kg de tissu placentaire contenant 65 % d'eau.

Le tissu extrait du pressoir est agité dans 500 l de citrate de sodium à 0,05 M pH 7,2 pendant 30 minutes à 10°C puis soumis au pressage pour éliminer la solution de lavage et récupérer le tissu. Un second lavage est effectué avec 500 l de citrate de sodium à 0,05 M pH 7,2 additionné de 30 g/l de NaCl. Un troisième lavage est effectué avec 500 l de citrate de sodium à 0,05 M pH 7,2. Le tissu ainsi lavé à pH neutre est ensuite soumis à trois séquences successives de lavage à pH acide et à 10°C :
- par 500 l d'acide citrique 0,05 M et à pH final ajusté à 2,8 par addition de HCl 2N ; l'agitation est de 30 minutes avant l'opération de pressage ;
- par 500 l d'acide formique 0,5 M pendant 15 h ;
- par 500 l d'acide citrique 0,05 M additionné de NaCl 20 g/l pendant 30 mn.

Le tissu placentaire ainsi lavé à pH acide a un aspect blanc qui témoigne d'une bonne élimination des pigments rouges du sang placentaire initial. Le poids obtenu est de 82 kg.

Il est soumis à une digestion enzymatique par la pepsine dans 500 l d'acide citrique 0,05 M pH 2,8 contenant 300 g de pepsine, pendant 15 h à 10°C. La suspension est alors diluée par addition de 500 l d'eau 10°C. Le pH est ajusté à 7,5 par addition de NaOH 4N, pour dénaturer la pepsine et en supprimer l'action protéasique. Après 15 h d'attente à 10°C, le résidu tissulaire qui contient l'essentiel des collagènes I est III non solubilisés est séparé par une centrifugeuse en continu (Westfalia KG 10006). Le poids de ce résidu est de 103 kg ; il peut être soumis à une deuxième digestion enzymatique identique à la première, suivie de l'extraction et de la séparation de chacun des collagènes I et III selon des procédés décrits dans la littérature.

Le surnageant correspondant à la première digestion enzymatique contient l'essentiel des collagènes et autres macromolécules solubles à pH neutre après l'action de la pepsine. Il contient en particulier du collagène de type IV.

Le pH du surnageant est ajusté à 5 avec HCl 2N et, après 15 h d'attente à 10°C, le précipité formé est éliminé par centrifugation en contenu sur centrifugeuse "Bactofuge" Alfa Laval.

Le surnageant recueilli, qui est très limpide, est ajusté à pH 7,5 par addition de NaOH 4N et à une concentration finale de 1,2 M en NaCl. Après 15 h à 16°C, le précipité de collagène formé est récpéré par centrifugation en continu sur centrifugeuse "Bactofuge".

Le précipité de 13 kg est ensuite dissous dans 600 l d'HCl 0,01 N puis le pH est ajusté à 7,5 par addition de NaOH 4N. Le précipité formé après 15 h d'attente à 4°C est éliminé sur centrifugeuse "Bactofuge" (poids obtenu 10 kg).

Le surnageant limpide est acidifié à pH 2,8 avec HCl 2N et additionné de NaCl jusqu'à une concentration finale de 0,6 M à 4°C.

Après 15 h, le précipité de collagène est recueilli sur centrifugeuse "Bactofuge". Le précipité dont le poids est de 6,5 kg a un aspect fluide. Il est additionné lentement de 7 l d'acétone, ce qui entraîne la formation de fibres de collagène qui l'on récupère par filtration sur tamis. Le lavage de ces fibres par plusieurs traitement à l'acétone permet, après séchage sous courant d'air tiède et stérile, d'obtenir 180 g de fibres sèches du produit final.

La dissolution dans de l'eau de ces fibres de collagène donne une solution acide.

Cette solution, amenée à 15 % en eau distillée, est chauffée à 45 - 60°C pendant 30 minutes. La solution parfaitement homogène et fluide est filtrée sur des membranes de porosité comprise entre 0,8 et 8 microns à cette même température et coulée dans un moule préalablement chauffé à 45-60°C pour une mise sous forme de lentilles ou d'implants ophtalmiques. L'ensemble est aussitôt refroidi à + 4°C durant 15 heures. Après démoulage, la lentille ou l'implant sont mis dans une solution de periodate de sodium 0,01 M à pH 7,5 pendant 15 heures à température ambiante. Ensuite, la lentille ou l'implant sont transférés dans un tampon glycocolle 20 g/l pH 7,5 ou éthanolamine 0,05 M et/ou dans une solution d'hydroborure de sodium 0,02 M pendant 3 heures à température ambiante.

Les implants ou lentilles obtenus sont parfaitement transparents, très souples et très hydrophiles et se révèlent d'une maniabilité plus facile, étant plus solides mécaniquement et d'une très bonne stabilité, résistant à un traitement thermique à 95°C pendant 1 heure dans un tampon à pH neutre, alors qu'un implant ou une lentille non traités au periodate de sodium sont très rapidement hydrolysés.

### EXEMPLE 2

Dans une solution acide de collagène parfaitement homogène et fluide à 15 % de l'exemple 1, on ajoute une solution d'acide periodique jusqu'à une concentration finale de 0,0001 M. Après homogénéisation et élimination des bulles, la solution est coulée dans un moule préalablement chauffé à 45-60°C. L'ensemble est aussitôt refroidi à + 4°C durant 15 heures.

Lors du démoulage, la lentille ou l'implant sont mis à tremper dans un tampon phosphate 0,01 M pH 7,5 NaCl 10 g/l durant 15 heures puis dans un tampon glycocolle 20 g/l pH 7,5 ou éthanolamine 0,05 M et/ou une solution d'hydroborure de sodium 0,02 M pendant 3 heures à température ambiante.

La lentille ou l'implant présentent les mêmes caractéristiques que celles décrites dans l'exemple 1.

### EXEMPLE 3

La lentille ou l'implant préparés suivant l'exemple 2 sont, lors du démoulage, mis à tremper dans une solution de periodate de sodium 0,01 M pH 7,5 pendant 15 heures à température ambiante, puis dans un tampon glycocolle 20 g/l NaCl 10 g/l pH 7,5 ou éthanolamine 0,05 M et/ou une solution d'hydroborure de sodium 0,02 M pendant 3 heures à température ambiante.

La lentille ou l'implant présentent les mêmes caractéristiques que celles décrites dans l'exemple 1.

### EXEMPLE 4

La lentille ou l'implant préparés suivant l'exemple 2 sont, lors du démoulage, mis à tremper dans une solution de glutaraldéhyde à 0,2 % pH 7,5 pendant 15 heures à température ambiante, puis dans un tampon glycocolle 20 g/l NaCl 10 g/l pH 7,5 ou éthanolamine 0,05 M et/ou une solution d'hydroborure de sodium 0,02 M pendant 3 heures à température ambiante

### EXEMPLE 5

Une solution de collagène humain (type III, I ou IV) ou de collagène acide bovin (type I) à 0,2 % en eau distillée est traitée au periodate de sodium 0,001 M pendant 2 heures à température ambiante. La solution est ensuite dialysée contre de l'acide chlorhydrique 0,01 N durant 20 heures.

Le pH de la solution est alors ajusté à 7,5 par addition de NaOH, 0,1 N ; après 10 mn à température ambiante, on a formation d'un gel qui, après lavage par des tampons glycocolle ou éthanolamine et équilibrage avec un milieu de culture, peut être utilisé pour la culture cellulaire ou comme produit de comblement de plaie.

On obtient ainsi à température ambiante des gels très stables résistant à des pH extrêmes (HCl 0,01 N) et très maniables. Ces gels sont obtenus aussi bien avec des collagènes acidosolubles ou solubilisés par traitement enzymatique ou alcalin, avec ou sans télopeptides.

### EXEMPLE 6

Un mélange volume à volume de collagène oxydé suivant l'exemple 5 et de collagène normal à 0,6 % en eau distillée dont le pH est ajusté à 7,5 avec NaOH 0,1 N se prend en gel en 10 mn à température ambiante.

### EXEMPLE 7

Un mélange de :
. 1 volume de collagène oxydé suivant l'exemple 5,
. 1 volume de collagène normal à 0,6 % en eau distillée,
. 0,2 volume de fibronectine plasmatique à 10 g/l se prend en gel en 10 mn à température ambiante et à pH neutre. Les proportions des différents réactifs peuvent être largement modifiées pour inclure plus de fibronectine si nécessaire.

### EXEMPLE 8

Une solution de collagène oxydée suivant l'exemple 5 est versée sous agitation dans un tampon phosphate 0,04 M pH 7,2 à 37°C. Il se forme aussitôt des fibrilles de collagène. Après 2 heures, ces fibrilles sont lavées en tampon glycocolle ou éthanolamine et/ou avec une solution d'hydroborure de sodium 0,02 M pendant 3 heures à température ambiante.

Ces fibrilles très stables ne se redissolvent pas en milieu acide et peuvent être utilisées comme produit de comblement pour les plaies ou pour l'adsorption de protéines (fibronectine, etc..).

### EXEMPLE 9

Les mélanges des exemples 6 et 7 sont versés sous agitation dans un tampon phosphate 0,04 M pH 7,5 à 37°C. Il se forme aussitôt des fibrilles de collagène. Après 2 heures, ces fibrilles sont lavées en tampon glycocolle ou éthanolamine et/ou avec une solution d'hydroborure de sodium 0,02 M pendant 3 h à température ambiante.

### EXEMPLE 10

Une solution de collagène oxydé suivant l'exemple 5 est versée dans une solution de fibronectine plasmatique à 0,01 g/l en tampon phosphate 0,04 M pH 7,5. Il se forme aussitôt des fibrilles de collagène englobant la fibronectine plasmatique.

### EXEMPLE 11

Une solution de collagène oxydé suivant l'exemple 5 est ajustée à pH 3 puis additionnée d'une solution de chlorure de sodium jusqu'à 1 M final. Après 15 heures, on récupère par centrifugation un précipité qui, après déshydratation par l'acétone 100 %, séchage sous courant d'air et broyage, donne une poudre de collagène insoluble possédant des groupements réactifs en vue d'un couplage ultérieur ou utilisable comme produit de comblement osseux.

### EXEMPLE 12

Une solution de collagène oxydé suivant l'exemple 5 est ajustée à pH 4 puis additionnée rapidement de fibronectine plasmatique 0,01 g/l et/ou de glycosaminoglycane 0,1 %. Après 2 heures de contact, on ajoute une solution de chlorure de sodium 1 M final. Après 15 heures, on récupère par centrifugation un précipité très solide pouvant être utilisé pour la réparation de lésions tissulaires.

### EXEMPLE 13

La solution de collagène oxydé suivant l'exemple 5 ou le mélange mis en oeuvre dans l'exemple 6 sont additionnés de glycérine 1,5 % en final puis coulés sur un support hydrophobe. Après séchage à température ambiante sous flux d'air stérile, on obtient un film. Après traitement de 30 minutes dans l'alcool 95 puis déshydratation dans un bain d'acétone 100 %, on obtient un film insoluble dont la souplesse, l'épaisseur et la résistance peuvent varier en fonction de la concentration en collagène et en glycérine.

### EXEMPLE 14

La solution de collagène oxydé suivant l'exemple 5 ou le mélange mis en oeuvre dans l'exemple 6 sont déposés goutte à goutte sur un mélange de 9 volumes d'acétone et 1 volume de NaOH 0,1 N. Chaque gouttelette se gélifie immédiatement et conserve définitivement sa forme sphérique. La suspension obtenue est ensuite lavée par une solution stérile physiologique pour éliminer l'acétone et neutraliser le pH de manière définitive.

Les billes ainsi obtenues peuvent être utilisées en culture cellulaire.

On peut, bien entendu, utiliser d'autres procédés pour réaliser de grandes quantités de microbilles de collagène. En particulier, on peut utiliser tous les procédés connus pour préparer des émulsions de microgouttelettes.

### EXEMPLE 15

. 1 volume de collagène oxydé suivant l'exemple 5,
. 1 volume de glycosaminoglycane à 0,1 % (acide chondroïtine 6 sulfate ou chondroïtine 4 sulfate, Heparan sulfate, Dermatan Sulfate, acide hyaluronique) dont le pH est ajusté à 7,5 avec NaOH 0,1 N permettent de réaliser des gels, des fibres ou des billes.

### EXEMPLE 16

Une solution de collagène oxydé à 0,5 % est mélangée volume à volume avec une solution de glycosaminoglycane à 0,2 %, le pH ajusté à 7,5 et le mélange aussitôt coulé dans un moule de manière à avoir une épaisseur d'environ 1 cm. Après 2 heures à température ambiante, le plateau est congelé et lyophilisé. On obtient des compresses plus résistantes à la biodégradation et utilisables comme produit de comblement.

### EXEMPLE 17

Un précipité salin de collagène, après déshydratation à l'acétone, séchage sous courant d'air et broyage, donne une poudre.

Cette dernière est reprise à 0,2 % dans une solution de NaIO4 0,001 M en alcool 95. Après 2 heures de contact sous agitation à température ambiante, la poudre est lavée sur toile Nylon en alcool 95 puis acétone 100 % avant séchage sous courant d'air et broyage.

Ces poudres devenues insolubles réagissent au réactif de Schiff. La poudre de collagène sans télopeptide (Trillagène de SADUC) reste soluble dans l'eau et se gélifie dés que le pH de la solution est ajusté à 7,5.

Ces poudres ainsi obtenues et la poudre obtenue dans l'exemple 11, qui sont insolubles, sont mélangées avec un collagène non oxydé et/ou des glycosaminoglycanes et/ou des antibiotiques pour former une pâte homogène utilisable comme produit de comblement osseux.

## Revendications

1. Procédé de réticulation du collagène en solution acqueuse, caractérisé en ce qu'il consiste à soumettre le collagène à une oxydation ménagée par une solution d'acide périodique ou de périodate, de sodium, à une concentration comprise entre 10⁻¹ et 10⁻⁴ M à température ambiante à pH acide, puis à procéder à une solidification du collagène.

2. Procédé selon la revendication 1, caractérisé en ce que la solidification est effectuée par poursuite du traitement à pH neutre ou alcalin.

3. Procédé selon la revendication 1, caractérisé en ce que l'étape de solidification s'effectue par chauffage.

4. Procédé de réticulation du collagène d'un gel de collagène déjà constitué, caractérisé en ce que l'on soumet le gel de collagène à une oxydation ménagée, par traitement par une solution d'acide périodique ou de périodate, de sodium, à une concentration comprise entre 10⁻¹ et 10⁻⁴ à un pH supérieur à 5, pendant 3 à 20 heures à température ambiante.

5. Procédé selon la revendication 4, caractérisé en ce que le pH est compris entre 5 et 8.

6. Procédé de réticulation de collagène solide sous forme de poudre ou de film, caractérisé en ce que l'on soumet le collagène à une oxydation ménagée par traitement par une solution d'acide périodique ou de périodate, de sodium, à une concentration comprise entre 10⁻¹ et 10⁻⁴ M à un pH suffisant pour empêcher sa resolubilisation, ledit pH étant compris, au moins dans un deuxième temps entre 5 et 8, pendant 3 à 20 heures, à température ambiante.

7. Procédé selon l'une quelconque des revendications précédentes, caractérisé en ce qu'il est suivi d'une étape de lavage ou de neutralisation.

8. Procédé selon la revendication 7, caractérisé en ce que le lavage est effectué par une solution de glycocolle.

9. Procédé selon la revendication 7, caractérisé en ce que le lavage est effectué par une solution d'éthanolamine.

10. Procédé selon la revendication 7, caractérisé en ce que le lavage est effectué par une solution d'hydroborure de sodium.

11. Procédé selon l'une quelconque des revendications 1 à 3, caractérisé en ce qu'il est éventuellement suivi par un tannage de surface.

12. Procédé selon la revendication 11, caractérisé en ce que le tannage de surface est effectué par une solution de periodate de sodium.

13. Procédé selon la revendication 11, caractérisé en ce que le tannage de surface est effectué par une solution de di- ou polyaldéhyde.

14. Procédé selon la revendication 12, caractérisé en ce que le tannage s'effectue avec une solution de periodate de sodium dont la concentration est comprise entre 0,1 et 0,001 M, à un pH compris entre 5 et 8, pendant 3 à 20 heures.

15. Procédé selon la revendication 12, caractérisé en ce que le tannage s'effectue avec une solution de di- ou polyaldéhyde dont la concentration est comprise entre 0,1 et 0,001 M, à un pH compris entre 5 et 8, pendant 3 à 20 heures.

16. Application des solutions collagéniques traitées par le procédé selon la revendication 1 à la préparation de poudres, de fibres, de billes ou de films.

17. Gel de collagène, caractérisé en ce qu'il est obtenu par application du procédé selon l'une des revendications 1 à 3, 7 à 15.

18. Collagène réticulé se présentant sous forme de films ou de poudres, caractérisé en ce qu'il a été soumis au traitement selon l'une quelconque des revendications 1, 4 et 8 à 12 6, 7 à 10.

19. Gel de collagène présentant des groupements réactifs utilisables pour un couplage ultérieur, caractérisé en ce qu'il est obtenu par application du procédé selon l'une quelconque des revendications 1 à 4, 7 à 15.

20. Collagène sous forme de films ou de poudres présentant en surface des groupements réactifs utilisables pour un couplage ultérieur, caractérisé en ce qu'il est obtenu par application du procédé selon la revendication 6.

21. Gel de collagène selon la revendication 19, caractérisé en ce que les groupements réactifs sont couplés avec des protéines, fibronectine, facteurs de croissance glycosaminoglycane enzymes, agents bactéricides ou bactériostatiques ou antibiotiques.

22. Collagène sous forme de films ou de poudres selon la revendication 20, caractérisé en ce que les groupements réactifs sont couplés avec des protéines, fibronectine, facteurs de croissance, glycosaminoglycane, enzymes, agents bactéricides ou bactériostatiques ou antibiotiques.

## Claims

1. A process for cross-linking collagen in aqueous solution, characterised in that it consists in subjecting the collagen to a controlled oxidation with a solution of periodic acid or of sodium periodate, at a concentration ranging between 10⁻¹ and 10⁻⁴ M at ambiant temperature at acidic pH, and then in carrying out a solidification of the collagen.

2. The process according to claim 1, characterised in that the solidification is carried out by continuing the treatment at neutral or alkaline pH.

3. The process according to claim 1, characterised in that the solidification step is carried out by heat.

4. A process for cross-linking collagen of an already constituted collagen gel, characterised in that the collagen gel is subjected to a controlled oxidation by treatment with a solution of periodic acid or of sodium periodate, at a concentration of between 10⁻¹ to 10⁻⁴ M, at a pH above 5, for 3 to 20 hours at ambiant temperature.

5. The process according to claim 4, characterised in that the pH is ranging between 5 and 8.

6. A process for cross-linking solid collagen in the form of powder or film, characterised in that collagen is subjected to a controlled oxidation by treatment with a solution of periodic acid or of sodium periodate, at a concentration of between 10⁻¹ to 10⁻⁴ M, at a pH which is sufficient to avoid its redissolution, said pH being, at least in a second stage, of between 5 and 8, for 3 to 20 hours at ambiant temperature.

7. The process according to any one of the preceding claims, characterised in that it is followed by a washing or neutralization step.

8. The process according to claim 7, characterised in that the washing is carried out using a solution of glycine.

9. The process according to claim 7, characterised in that the washing is carried out using a solution of ethanolamine.

10. The process according to claim 7, characterised in that the washing is carried out using a solution of sodium borohydride.

11. The process according to any one of the claims 1 to 3, characterised in that it may be followed by a surface tanning.

12. The process according to claim 11, characterised in that the surface tanning is carried out using a solution of sodium periodate.

13. The process according to claim 11, characterised in that the surface tanning is carried out using a solution of di- or polyaldehyde.

14. The process according to claim 12, characterised in that the tanning is carried out using a solution of sodium periodate the concentration of which is between 0.1 and 0.001 M, at a pH of between 5 and 8, for 3 to 20 hours.

15. The process according to claim 12, characterised in that the tanning is carried out using a solution of di- or polyaldehyde the concentration of which is between 0.1 and 0.001 M, at a pH of between 5 and 8, for 3 to 20 hours.

16. An application of the collagenic solutions treated by the process according to claim 1, to the preparation of powders, fibers, spheres or films.

17. A collagen gel characterised in that it is obtained by application of the process according to one of the claims 1 to 3, 7 to 15.

18. A cross-linked collagen in the form of films or powders, characterised in that it has been subjected to the treatment according to any one of the claims 1, 4, 6, 7 to 10 and 8 to 12.

19. A collagen gel with reactive groups useable for subsequent coupling, characterised in that it is obtained by application of the process according to any one of the claims 1 to 4, 7 to 15.

20. A collagen in the form of films or powders with reactive groups in surface useable for subsequent coupling, characterised in that it is obtained by application of the process according to claim 6.

21. The collagen gel according to claim 19, characterised in that the reactive groups are coupled with proteins, fibronectin, growth factors, glycosaminoglycans, enzymes, bactericidal or bacteriostatic agents, or antibiotics.

22. The collagen in the form of films or powders according to claim 20, characterised in that the reactive groups are coupled with proteins, fibronectin, growth factors, glycosaminoglycans, enzymes, bactericidal or bacteriostatic agents, or antibiotics.

## Patentansprüche

1. Verfahren zur Vernetzung von Kollagen in wäßriger Lösung, dadurch gekennzeichnet, daß es darin besteht, das Kollagen einer Oxidation zu unterziehen, die mit einer Periodsäure- oder Natriumperiodat-Lösung bei einer Konzentration zwischen 10⁻¹ und 10⁻⁴ M eingeschlossen bei Umgebungstemperatur bei saurem pH durchgeführt wird, dann eine Verfestigung des Kollagens vorzunehmen.

2. Verfahren nach Anspruch 1, dadurch gekennzeichnet, daß die Verfestigung durch Fortführung der Behandlung bei neutralem oder alkalischem pH bewirkt wird.

3. Verfahren nach Anspruch 1, dadurch gekennzeichnet, daß der Schritt der Verfestigung durch Erwärmen bewirkt wird.

4. Verfahren zur Vernetzung von Kollagen aus einem bereits gebildeten Kollagengel, dadurch gekennzeichnet, daß man das Kollagengel einer Oxidation unterzieht, die durch Behandlung mit einer Periodsäure- oder Natriumperiodat-Lösung bei einer Konzentration zwischen 10⁻¹ und 10⁻⁴ eingeschlossen bei einem pH oberhalb von 5 über 3 bis 20 Stunden bei Umgebungstemperatur durchgeführt wird.

5. Verfahren nach Anspruch 4, dadurch gekennzeichnet, daß der pH zwischen 5 und 8 eingeschlossen ist.

6. Verfahren zur Vernetzung von festem Kollagen in Form von Pulver oder eines Films, dadurch gekennzeichnet, daß man das Kollagen einer Oxidation unterzieht, die durch Behandlung über 3 bis 20 Stunden bei Umgebungstemperatur mit einer Periodsäure- oder Natriumperiodat-Lösung bei einer Konzentration zwischen 10⁻¹ und 10⁻⁴ M eingeschlossen bei einem pH durchgeführt wird, der ausreicht, um dessen Wiederauflösung zu verhindern, wobei der pH mindestens zu einem zweiten Zeitpunkt zwischen 5 und 8 eingeschlossen ist.

7. Verfahren nach irgendeinem der vorangehenden Ansprüche, dadurch gekennzeichnet, daß darauf ein Wasch- oder Neutralisationsschritt folgt.

8. Verfahren nach Anspruch 7, dadurch gekennzeichnet, daß das Waschen durch eine Glykokoll-Lösung bewirkt wird.

9. Verfahren nach Anspruch 7, dadurch gekennzeichnet, daß das Waschen durch eine Ethanolamin-Lösung bewirkt wird.

10. Verfahren nach Anspruch 7, dadurch gekennzeichnet, daß das Waschen durch eine Natriumborhydrid-Lösung bewirkt wird.

11. Verfahren nach irgendeinem der Ansprüche 1 bis 3, dadurch gekennzeichnet, daß darauf gegebenenfalls eine Gerbung der Oberfläche folgt.

12. Verfahren nach Anspruch 11, dadurch gekennzeichnet, daß das Gerben der Oberfläche durch eine Natriumperiodat-Lösung bewirkt wird.

13. Verfahren nach Anspruch 11, dadurch gekennzeichnet, daß die Gerbung der Oberfläche durch eine Di- oder Polyaldehyd-Lösung bewirkt wird.

14. Verfahren nach Anspruch 12, dadurch gekennzeichnet, daß die Gerbung über 3 bis 20 Stunden mit einer Natriumperiodat-Lösung, deren Konzentration zwischen 0,1 und 0,001 M eingeschlossen ist, bei einem pH zwischen 5 und 8 eingeschlossen bewirkt wird.

15. Verfahren nach Anspruch 12, dadurch gekennzeichnet, daß die Gerbung über 3 bis 20 Stunden mit einer Di- oder Polyaldehyd-Lösung, deren Konzentration zwischen 0,1 und 0,001 M eingeschlossen ist, bei einem pH zwischen 5 und 8 eingeschlossen bewirkt wird.

16. Verwendung von Kollagenlösungen, die durch das Verfahren nach Anspruch 1 behandelt worden sind, bei der Herstellung von Pulvern, Fasern, Klötzchen oder Filmen.

17. Kollagengel, dadurch gekennzeichnet, daß es durch Verwendung des Verfahrens nach irgendeinem der Ansprüche 1 bis 3, 7 bis 15 erhalten wird.

18. Vernetztes Kollagen, das in Form von Filmen oder Pulvern vorliegt, dadurch gekennzeichnet, daß es einer Behandlung gemäß irgendeinem der Ansprüche 1, 4 und 8 bis 12, 6, 7 bis 10 unterzogen worden ist.

19. Kollagengel, das reaktive Gruppen aufweist, die für eine spätere Kupplung verwendbar sind, dadurch gekennzeichnet, daß es durch Anwendung des Verfahrens gemäß irgendeinem der Ansprüche 1 bis 4, 7 bis 15 erhalten worden ist.

20. Kollagen in Form von Filmen oder Pulvern, das auf der Oberfläche reaktive Gruppen aufweist, die für eine spätere Kupplung verwendbar sind, dadurch gekennzeichnet, daß es durch Anwendung des Verfahrens gemäß Anspruch 6 erhalten worden ist.

21. Kollagengel gemäß Anspruch 19, dadurch gekennzeichnet, daß die reaktiven Gruppen mit Proteinen, Fibronektin, Wachstumsfaktoren, Glykosaminoglykan, Enzymen, bakteriziden oder bakteriostatischen Mitteln oder Antibiotika gekuppelt sind.

22. Kollagen in Form von Filmen oder Pulvern nach Anspruch 20, dadurch gekennzeichnet, daß die reaktiven Gruppen mit Proteinen, Fibronektin, Wachstumsfaktoren, Glykosaminoglykan, Enzymen, bakteriziden oder bakteriostatischen Mitteln oder Antibiotika gekuppelt sind.
